# EUROPEAN PATENT APPLICATION

(11) **EP 0 943 681 A1**
(43) Date of publication of application: **22.09.1999**
(21) Application number: 98870019.1
(22) Date of filing: 22.01.1998
(51) Int. Cl.: C12N 15/11, A61K 39/112, C07K 14/255

(54) **Live attenuated salmonella vaccine**

(71) Applicant: VRIJE UNIVERSITEIT BRUSSEL, 1050 Brussel (BE)
(72) Inventor: Gubbels, Elina Frieda Irena Albert, 2100 Antwerpen-Deurne (BE); De Greve, Henri Marcel Jozef, 1060 Brussel (BE); Hernalsteens, Jean-Pierre Ernest Clément, 1150 Sint-Pieters-Woluwe (BE)
(74) Representative: Van Malderen, Joelle

(57) **Abstract**

The present invention is related to a vaccine for inducing an immune response to a *Salmonella* strain in an animal, including a human, characterised in that it comprises a pharmaceutically acceptable carrier and a genetically modified *Salmonella* strain which is in an amount effective to produce an immune response in said animal, including human, and comprises a modification in its wild type DNA sequence SEQ ID NO 01, any of the DNA sequences from the same operon as SEQ ID NO 01 and/or any regulatory sequence of any of the said DNA sequences.

## Description

### Field of the invention

The present invention relates to a pharmaceutical composition, such as a vaccine, for administration to animals, including humans, said pharmaceutical composition being able to produce an immune response against infection induced by *Salmonella* strains and/or other pathogens.

It further relates to the preparation process and to the use of said pharmaceutical composition.

### State of the art

*Salmonella* is an important pathogen of both humans and livestock. In recent years, a steady increase has been noted of the incidence of human nontyphoidal salmonellosis, reflecting changes in animal husbandry, the mechanisation of food processing (particularly of eggs) and the mass distribution of food (Falkow S. and Mekalanos J. : The enteric Bacilli and Vibrios. In: *Microbiology*, edited by Davis, B.D., Dulbecco, R., Eisen, H.N. and Ginsberg, H.S. Philadelphia: Lippincott Co., 1990, p. 561-587). In particular, the number of human infections due to *Salmonella enteritidis* contamination of eggs and poultry meat has increased dramatically. Data of the National Reference Laboratory show that these constituted about one half of the total number of human salmonelloses in Belgium in 1996. The problem is exacerbated by the fact that the infected chickens often show no clear symptoms, while the germ can cause a serious and potentially lethal disease in humans.

The ubiquitous presence of *Salmonella* in nature complicates the control of the disease just by detection and eradication of infected animals. Therefore vaccination of farm animals is often considered as the most effective way to prevent zoonoses caused by *Salmonella*. Different strategies were tested for the production of *Salmonella* vaccines. Inactivated cells are often not effective as vaccines. This can be explained by the fact that numerous virulence genes are tightly regulated and therefore not expressed under *in vitro* culture conditions. A more promising alternative is the use of living *Salmonella* cells, with a mutation in a gene essential for virulence, as attenuated living vaccines. Such vaccines often simultaneously elicit effective humoral, local and cellular immunity. They have the additional advantage that an oral administration is possible. This avoids the labour of injecting individual animals and is an important advantage in, poultry production.

The use of several types of *Salmonella* mutants as potential live attenuated vaccines has been described, including among others:
- auxotrophic mutants, such as the *aro* mutants disclosed in patent US-5,643,771;
- mutants deficient in the production of adenylate cyclase and the cyclic AMP receptor protein, as disclosed in patent US-5,389,368
- mutants with an altered expression of outer membrane proteins, as disclosed in patent US-5,527,529
- reverse mutants of streptomycin dependant mutants, as disclosed in patent US-4,350,684
- mutants in which the regulation of gene expression is altered by a mutation in the *pho*P/*pho*Q regulatory system, such as those disclosed in patents US-5,424,065 and US-5,674,736
- strains carrying one or more unidentified mutations, such as these obtained by *in vitro* passage through phagocytic cells (as disclosed in the US Patent US-5,436,001) or after mutagenesis (as disclosed in patent US-3,856,935).

The use of these strains as vaccines was often hampered by problems such as insufficient immunogenicity or excessive residual virulence.

In a number of vaccine strains, the molecular basis of the attenuating mutation is not known. For example in the US Patent US-5,436,001, a live avirulent *Salmonella choleraesuis* vaccine is disclosed. The vaccine is obtained by passing the wild-type bacteria through phagocytic cells such as macrophages or polymorphonuclear leukocytes, a sufficient number of times until the bacteria becomes avirulent to the animal host. However, said vaccine is very limited in ,its use, since it has to be obtained from scratch with every new strain one wants a vaccine for. Moreover, since the exact nature (genotypic and/or phenotypic) of the strain modification is not explicitly known, it is not certain that the obtained strain will remain avirulent, and that its modification is transferable to other strains. There is also no routine test allowing the distinction of the vaccine strain from related virulent salmonellae.

### Aims of the invention

A first aim of the invention is to provide a pharmaceutical composition such as a vaccine able to produce an immune response against a *Salmonella* strain in animals, including humans, and which does not present the drawbacks of the state of the art.

A second aim of the invention is to provide a pharmaceutical composition such as a vaccine able to produce an immune response against pathogenic agents other than *Salmonella* infecting animals, including humans, and which does not present the drawbacks of the state of the art.

Another aim of the invention is to identify sequences, involved in virulence, in *Salmonella* strains and to provide a new preparation method of an avirulent *Salmonella* strain.

### Summary of the invention

The invention refers to a vaccine for inducing an immune response to a *Salmonella* strain in an animal, including a human, said vaccine comprising a pharmaceutically acceptable carrier and one or more genetically modified *Salmonella* strain(s) in an amount effective to produce said immune response (humoral, local and/or cellular immune response) and wherein said genetically modified *Salmonella* strain comprises a modification in its wild type DNA sequence SEQ ID NO 01, its complementary strand, or in a homologous sequence.

Indeed, the Inventors have discovered that a *Salmonella* strain comprising a modification in its wild type DNA sequence SEQ ID NO 01 and/or its complementary strand becomes avirulent.

Said isolated and/or purified wild type DNA sequence SEQ ID NO 01 is identified in the enclosed sequence listing, and the genetic modification of said isolated and/or purified wild type DNA sequence SEQ ID NO 01 is preferably an insertion and/or a deletion of at least one nucleotide in said DNA sequence.

The Inventors have discovered unexpectedly that it is possible to reduce the "virulence" of a *Salmonella* strain by a genetic modification of said wild type DNA sequence. This sequence directly or indirectly promotes the virulence of *Salmonella* strains.

The "virulence" of the pathogen (*Salmonella* strain according to the invention) means the induction in an animal (including human) of infection and symptoms (salmonellosis) due to *Salmonella* contamination.

It is clear that genetic modifications in any DNA sequence belonging to the same operon than the DNA sequence as in SEQ ID NO 01, including its complementary strand and/or genetic modifications in any regulatory sequence of any of the said DNA sequences, may also result in a reduction in virulence of *Salmonella* strains as described above.

In the vaccine according to the invention, the pharmaceutically acceptable carrier can be any compatible non-toxic substance suitable for administering the composition (vaccine) according to the invention.

The pharmaceutically acceptable carriers according to the invention suitable for oral administration are the ones well known by the person skilled in the art, such as tablets, coated or non-coated pills, capsules, solutions or syrups. Other adequate pharmaceutical carriers or vehicles may vary according to the mode of administration (intravenous, intramuscular, parenteral, etc.).

The vaccine according to the invention may comprise also adjuvants well known by the person skilled in the art which may increase or regulate the humoral, local and/or cellular response of the immune system against *Salmonella* strains, other pathogenic agents or other epitopes. The vaccine according to the invention is prepared by the methods generally applied by the person skilled in the art for the preparation of a vaccine wherein the percentage of the active compound/pharmaceutically acceptable carrier can vary within very large ranges, only limited by the tolerance and the level of acquaintance of the patient to the vaccine. The limits are particularly determined by the frequency of administration.

Advantageously, the genetically modified *Salmonella* strain in the vaccine according to the invention may also comprise an isolated (and preferably purified) nucleotide sequence encoding a *Salmonella*-foreign antigen and said genetically modified *Salmonella* strain is present in the vaccine in an amount effective to induce an immune response to laid *Salmonella*-foreign antigen.

The isolated (and preferably purified) nucleotide sequences encoding *Salmonella*-foreigner antigens are the ones well known by the person skilled in the art and described in the scientific literature and known to induce an immune response against pathogenic agents such as bacteria, viruses or eukaryotic pathogenic agents which may induce infectious diseases in animals, including humans, or against other epitopes or epitope-bearing entities such as tumor antigens or portions thereof or a combination thereof, hormones, allergens, toxins, etc.

Preferably, the genetically modified *Salmonella* strain according to the invention is selected from the group consisting of the following *Salmonella* : *Salmonella enteritidis* (preferably *Salmonella enteritidis* EZ1263 having the deposit number LMGP-18112), *Salmonella typhimurium, Salmonella choleraesuis, Salmonella dublin, Salmonella paratyphi, Salmonella typhi, Salmonella hadar, Salmonella infantis, Salmonella montevideo* and *Salmonella senftenberg.*

Another aspect of the present invention is related to a (preferably virulent) isolated or synthetic nucleotide sequence having at least 40 % homology with the wild type DNA sequence SEQ ID NO 01 or its complementary strand.

Another aspect of the present invention is related to a (preferably virulent) isolated or synthetic amino acid sequence having at least 30% homology with the wild type amino sequence SEQ ID NO 02.

The term "isolated" means that the material is removed from its original environment (e.g., the natural environment if it is naturally occurring).

The variant of the SEQ ID NO 1 may be a naturally occurring allelic variant of SEQ ID NO 1 or a non-naturally occurring variant of SEQ ID NO 1.

As known in the art, an allelic variant is an alternate form of a sequence which may have a substitution, deletion or addition of one or more nucleotides and/or amino acids which preferably does not substantially alter the function of the encoded polypeptide.

A "virulent" genetic sequence is a nucleotide or amino acid sequence that is important for the infectious ability of a pathogen.

Said sequences may present an industrial application in the field of diagnostic (identification of various virulent increasing virulent salmonella strains) or for development of an avirulent vaccine comprising said sequences.

A further aspect of the present invention concerns a preparation method of an avirulent *Salmonella* strain, comprising the steps of:
- identifying a "virulent" nucleotide sequence in the genome of a *Salmonella* strain by any method based on the use of nucleotide sequence SEQ ID NO 01 or the complementary strand thereof, such as hybridisation or amplification by the polymerase chain reaction with a probe or primers having at least 12 nucleotides and which shows at least 10 identical nucleotides with a corresponding portion of SEQ ID NO 01 or its complementary strand or which shows more than 50% homology with a corresponding portion of SEQ ID NO 01 or its complementary strand.
- inducing a modification in said "virulent" nucleotide sequence in order to obtain an avirulent or less virulent nucleotide sequence, and
- recovering an obtained avirulent *Salmonella* strain having said modification in its "virulent" sequence.

In a preferred embodiment of the present invention, said hybridization is obtained under standard stringent hybridization conditions or which would hybridize for the redundancy of the genetic code.

Exemplary stringent hybridization conditions are as follows: hybridization at 42°C in 50% formamide, 5X SSC, 20 mM sodium phosphate, pH 6.8 washing in 0.2X SSC at 55°C. It is understood by those skilled in the art that variation in these conditions occurs based on the length and GC nucleotide content of the sequences to be hybridized. Formulas standard in the art are appropriate for determining exact hybridization conditions. *See Sambrook et al*., §§ 9.47-9.51 *in* Molecular Cloning: A Laboratory Manual, Cold spring Harbor Laboratory Press, Cold Spring Harbor, New York (1989).

The present invention is also related to a method for inducing an immune response to a *Salmonella* strain in an animal, including a human, comprising administering a pharmaceutical composition preferably comprising a pharmaceutically acceptable carrier and a live, genetically modified *Salmonella* strain to said animal, wherein said genetically modified *Salmonella* strain is in an amount effective to produce an immune response and wherein said genetically modified *Salmonella* strain comprises a modification in its wild type DNA sequence SEQ ID NO 01 and/or its complementary strand. Said genetically modified *Salmonella* strain is preferably administered in a pharmaceutically acceptable carrier.

In the method according to the invention, the modification of the "virulent" sequence is preferably obtained by an insertion and/or a deletion of at least one nucleotide in said nucleotide sequence. Said insertion or deletion is preferably obtained by homologous recombination with an engineered nucleotide sequence, comprising said insertion or deletion.

The present invention is also related to the use of the pharmaceutical composition, preferably the vaccine according to the invention for the preparation of a medicament for inducing an immune response to a *Salmonella* strain in an animal, including a human, preferably for inducing therapeutic and/or protective properties against a *Salmonella* strain and avoid salmonellosis diseases.

Advantageously, said immune response is an effective humoral, local and/or cellular immune response.

The present invention will be described in details in the following examples, in reference to the following figures which are presented as illustration of the various embodiments of the present invention without limiting its scope.

### Short description of the drawings

- Figure 1: represents the result of an ELISA test, showing that antibodies directed against *Escherichia coli* F17 fimbriae are produced after infection of mice with *S. enteritidis EZ1263* producting these fimbrae.
- Figure 2: represents the result of an ELISA test, showing antibodies directed against *S*. *enteritidis* lipopolysaccharides are produced after infection of mice with *S. enteritidis EZ1263* producing *Escherichia coli* F17 fimbrae.

### Examples

### Example 1 : Construction of the attenuated S. enteritidis mutant EZ1263

The *S. enteritidis* phage type 4 strain 76Sa88 (a clinical isolate from a chicken, obtained from the National Institute of Veterinary Research, Belgium) was used for the isolation of attenuated transposon insertion mutants. To facilitate the selection, the spontaneous rifampicin resistant mutant 76Sa88Rif^{R} was first isolated by plating samples of an overnight culture of 76Sa88 in LB medium (Miller, J.H. *Experiments in Molecular Genetics,* Cold Spring Harbor, New York: Cold Spring Harbor Laboratory, 1972. pp. 1-466) onto LB plates containing 100 mg/l rifampicin. Oral infection of Balb/c mice confirmed that the rifampicin resistance mutation of the strain 76Sa88Rif^{R} does not affect its virulence.

Mutants of 76Sa88Rif^{R} were isolated by insertion mutagenesis using the transposon miniTn*5lacZ1*, that generates transcriptional fusions with the β-galactosidase gene *lac*Z (de Lorenzo V., Herrero M., Jakubzik U., Timmis K.N., J. Bacteriol. 172 (11): 6568-6572, 1990). This system allows the identification of insertion mutations in genes that show a particular transcriptional regulation pattern. The transposon miniTn*5lacZ1* is harboured on the suicide plasmid pUT that is unable to replicate in *Salmonella*. Transposon miniTn*5lacZ1* insertion mutants were obtained by conjugation of *E. coli* S17-1(λ*pir*) (Simon R., Priefer U., and Pühler A., Biotechnology 1, 784-791, 1983), harbouring the pUT plasmid containing the miniTn*5lacZ1* transposon, with 76Sa88Rif^{R} on LB medium. The insertion mutants were selected subsequently on LB plates with 100 mg/l kanamycin (marker of miniTn*5lacZ1*) and 100 mg/l rifampicin. After colony purification, the mutants were tested for growth on LB medium with 100 mg/l carbenicillin (marker of pUT) to confirm the loss of the suicide plasmid. The carbenicillin-sensitive clones were cultured in 96-well microplates and replicated on different media simulating the conditions in the host. These media also contained the β-galactosidase substrate 5-bromo-4-chloro-3-indolyl-β-D-galactopyranoside (X-gal), that produces a blue precipitate upon hydrolysis. In one of the insertion mutants, strain EZ1263, β-galactosidase expression was induced by culture on the defined medium Minimal A (Miller, 1972), by culture at low pH (LB medium, buffered at pH 5.5 with 0.1 M MES (2-[N-Morpholino]ethanesulfonic acid) and by the iron chelator dipyridyl (0.2 mM). This induction pattern suggested that the corresponding gene might also be expressed *in vivo* after infection of the animal host, as is predicted for a virulence factor. Therefore the pathogenicity and immunogenicity of the strain EZ1263 were tested in mice (See following examples 3, 4, 5, 7 and 8).

A deposit has been made according to the Budapest Treaty for the micro-organism *Salmonella enteritidis* EZ1263 under deposit number LMG P-18112 at the BCCM/LMG Culture Collection, Laboratorium voor Moleculaire Biologie, Ledeganckstraat 35, B-9000 Gent (Belgium)

### Example 2: Identification of the mutation causing the attenuation of S. enteritidis EZ1263

To analyse the mutated gene in EZ1263, total genomic DNA of the mutant was prepared (Ausubel F.M., Brent R., Kingston R.E., Moore D.D., Seidman J.D., Smith J.A. and Struhl K. *Current Protocols in Molecular Biology*. Wiley Interscience, 1987), digested to completion with the restriction enzyme *Taq*I and circularised under conditions that favour intra-molecular ligation (1 µg DNA in a total reaction volume of 200 µl, using 0.1 unit of T4 DNA ligase, incubation overnight at 4 °C). This circular DNA was immediately used as template in IPCR amplification. PCR primers were designed according to the fusion fragment obtained after *Taq*I digestion, containing the first 836 bp of the *lacZ* coding sequence. Four *lacZ*-specific primers, forming two nested pairs, were synthesised (see Table 1).

**Table 1**

| **Synthetic oligonucleotides used as PCR primers** | | | | |
|---|---|---|---|---|
| Primer | Sequence from 5' to 3' | Restriction site | Position | SEQ ID NO |
| lacZ1* | GGGAATTCAAAGCGCCATTCGCCATTCAG | *Eco*RI | 467-1446 | 3 |
| lacZ2* | GGAAGCTTTATGGCAGGGTGAAACGCAGG | *Hind*III | 2062-2085 | 4 |
| lacZ3* | CGTCTAGACGTTTTCCCAGTCACGAC | *Xba*I | 1340-1324 | 5 |
| lacZ4* | GCGGATCCTTTCGGCGGTGAAATTATC | *Bam*HI | 2103-2123 | 6 |
| 1263-1** | ACAGACGATTTTTCTCTA | - | - | 7 |
| 1263-2B** | CGCCCCATTAAAGGCTAT | - | - | 8 |

| | | | | |
|---|---|---|---|---|
| * *lacZ*-specific PCR primers with their incorporated restriction enzyme sites (underlined) and their position in the *E. coli lacZ* gene (ECOLAC, Accession number: J01636) | | | | |
| ** SEQ ID 01-specific primers | | | | |

The IPCR reaction mixture consisted of 0.4 µM primer lacZ1, 0.4 µM primer lacZ2, template DNA (5 µl intra-molecular ligation mixture), 200 µM of each dNTP and 0.1 unit Super*Tth* Taq DNA polymerase (H.T. Biotechnology) in 50 µl Tth buffer (H.T. Biotechnology). The reaction conditions were as follows: three initial cycles of 94 °C for 1 min, 53 °C for 1 min and 72 °C for 1 min, followed by 35 cycles of 94 °C for 30 sec, 53 °C for 30 sec and 72 °C for 1 min. Gel-purified IPCR products (Jetsorb Gel Extraction kit, Genomed) were reamplified with nested primers lacZ3 (0.4 µM) and lacZ4 (0.4 µM) in a 50 µl reaction mixture as previously described. Twenty-five re-amplification cycles were done at 94 °C for 30 sec, 53 °C for 30 sec and 72 °C for 1 min. The IPCR and PCR amplifications were successful, using a Koch Light N.B.S. TC-1 thermocycler or a Perkin-Elmer GeneAmp PCR system 9600.

After re-amplification, the PCR products were directly sequenced using the Sequenase™ PCR Product Sequencing Kit (USB/Amersham). Starting with 0.2-0.5 pmol template, DNA sequencing was performed with the pUC forward primer provided in the kit, as well as with the lacZ4 primer.

The obtained PCR product has an estimated size of 650 bp. The 317 nucleotides upstream of the transposon were sequenced using the pUC forward primer and the lacZ4 primer. A search for homologous sequences in the bacterial DNA database was done with the obtained nucleotide sequences, using the FastA programme (Sequence Analysis Software Package, Genetics Computer Group, Inc.) and revealed no homologies with any of the known sequences.

To obtain the wild-type sequence corresponding to the mutated gene in EZ1263, a cosmid library of *S*. *enteritidis* genomic DNA was screened, using the re-amplification PCR-product as a probe specific for the mutated gene. The construction of the cosmid library and the procedure for colony hybridisation were described (Woodward M.J., Allen-Vercoe E., Redstone J.S., Epidemiol Infect 117 (1): 17-28, 1996). Fixation of the DNA on the Hybond N membrane was done by UV cross-linking. The PCR product obtained for mutant EZ1263 was used as a probe for hybridisation, after re-amplification with primers lacZ3 and lacZ4 and purification from an agarose gel (Jetsorb Gel Extraction kit, Genomed). The Ready To Go DNA labelling Beads (Pharmacia Biotech) were used to radio-label 25-30 ng of the purified PCR fragment. Unincorporated radioactive nucleotides were separated from the labelled probe with a ProbeQuant™ G-50 Micro Column (Pharmacia Biotech). During the labelling procedure, the hybridisation membranes were prehybridised in RapidHyb (Amersham) buffer at 65 °C (30-60 min.). The labelled probe was added to the membranes and hybridisation continued for 2-3 h. at 65 °C. After hybridisation, the membranes were washed: once 20 min. in 0.3 M NaCl, 0.03 M Na₃-citrate, 0.1 % SDS at room temperature and twice 15 min. at 65 °C in 0.03 M NaCl, 3 mM Na₃-citrate, 0.1 % SDS. The signal was detected by putting an X-ray film on top of the membrane and incubating at room temperature.

Four cosmid clones showed hybridisation with the EZ1263-probe and were sorted from the cosmid library stock. Cosmid DNA was prepared using the Qiagen Plasmid midikit as described in the manual. The obtained DNA was digested with several restriction endonucleases, separated by agarose gel electrophoresis and used for Southern blotting by standard procedures. Prehybridisation, hybridisation and labelling of the probe was carried out as described above. This hybridisation confirmed the homology of clones 3B7 and 4F9 with the EZ1263-probe. Furthermore it showed that the probe hybridised with a 1.7 kb *Pst*I fragment, a 4.5 kb *Eco*RV fragment and a 8 kb *Bam*HI fragment. For the endonucleases *Bgl*II, *Eco*RI and *Hind*III the size of the hybridising fragment was larger than 12 kb.

The 1.7 kb *Pst*I fragment was cloned into the *Pst*I site of the vector pUC19 using standard techniques. This resulted in the vector pGV4357. Several deletions in the insert were constructed to facilitate sequencing of the complete 1.7 kb *Pst*I fragment. Sequence analysis was carried out using the SequiTherm Cycle sequencing kit (Epicentre Technologies) or the Pharmacia ALF automatic DNA sequencer or the ThermoSequenase radio-labelled terminator cycle sequencing kit (Amersham). The transposon insertion in mutant EZ1263 is located in an open reading frame of 771 base pairs, encoding a prospective protein of 257 amino acids.

Screening of the nucleotide sequence databases with the nucleotide sequence SEQ ID NO 01, using the programmes BLASTN (Altschul S.F., Gish W., Miller W., Myers E.W., and Lipman D.J., J. Mol. Biol. 215, 403-10, 1990) and FastA (Sequence Analysis Software Package, Genetics Computer Group, Inc.), confirmed the absence of *Salmonella* sequences with a significant homology. The most related nucleotide sequence detected by BLASTN was the *van*X gene of transposon Tn*1546* of *Enterococcus faecalis,* encoding a D-alanyl-D-alanine dipeptidase involved in vancomycin resistance (accession number M97297). Alignment of the coding sequence of *van*X with SEQ ID O1 (using the PCgene programme NAlign with open gap cost 50 and unit gap cost 10) resulted in 298 identical nucleotides (38.6 %). Alignment of the amino acid sequence of VanX with SEQ ID O2 (using the PCgene programme PAlign, comparison matrix: Dayhoff MDM-78 with open gap cost 200 and unit gap cost 100) resulted in 71 identical residues (27.7 %) and 38 similar residues.

Within the *Enterobacteriaceae*, the highest degree of sequence identity was found using TBLASTX (that compares a nucleic acid sequence translated in the six translation frames against a nucleic acid database translated sequence by sequence in the six translation frames) with an *E. coli* sequence (accession number AE000245) that was determined as part of the *E. coli* Genome Project. The homologous sequence encodes a putative protein, called f193, of 193 amino acids that is 41 % identical (22 gaps) to 154 residues from D-alanyl-D-alanine dipeptidase VanX. Alignment of the coding sequence of f193 with SEQ ID O1 (using the PCgene programme NAlign with open gap cost 50 and unit gap cost 10) resulted in 259 identical nucleotides (33.5 %). Alignment of the amino acid sequence of f193 with SEQ ID O2 (using the Pcgene programme PAlign, comparison matrix: Dayhoff MDM-78 with open gap cost 200 and unit gap cost 100) resulted in 62 identical residues (24.2 %) and 29 similar residues.

### Example 3 : Induction of protective immunity against S. enteritidis after intra-peritoneal vaccination of mice with S. enteritidis EZ1263

*S. enteritidis EZ1263* was cultured overnight at 37 °C in LB medium, spun down and resuspended in PBS (1.5 mM KH₂PO₄, 10 mM Na₂HPO₄, 140 mM NaCl, 3 mM KCl, pH 7.2). Intraperitoneal injection of 2.1 10² colony forming units of these EZ1263 bacteria in 5-6 week old female Balb/c mice did not produce perceptible disease symptoms, while all control mice injected with 2.3 10² colony forming units of the wild type *S. enteritidis* 76Sa88 under identical conditions were killed by the infection (see Table 2).

**Table 2**

| **Results of intra-peritoneal infection of mice with wild type *S. enteritidis* 76Sa88 or mutant EZ1263** | | | |
|---|---|---|---|
| Strain | Dose (cfu) | Number of surviving mice | Dead days after inoculation |
| EZ1263 | 2.1 10² | 3/3 | / |
| 76Sa88 | 2.3 10² | 0/3 | 9, 9, 9 |
| Negative control | 0 | 3/3 | / |

The mice that were intraperitoneally injected with strain EZ1263 were submitted to an oral challenge of wild type *S. enteritidis* 76Sa88 after 34 days. The germs were cultured overnight in LB broth at 37 °C, spun down and resuspended in milk. 2.8 10⁸ colony forming units (in 50µl milk) were applied using a micropipette. This corresponds with about 10⁵ LD₅₀ units. All of the injected mice survived the, challenge infection without observable disease symptoms, while all of non-injected control mice that were orally challenged under identical conditions were killed (see Table 3).

**Table 3**

| **Protection against oral infection with wild type** ***S. enteritidis*** **76Sa88 after intraperitoneal (Ip) infection with mutant EZ1263** | | | |
|---|---|---|---|
| Previous IP. Infection | Dose of 76Sa88 (cfu) | Number of surviving mice | Dead days after inoculation |
| EZ1263 | 3.3 10⁸ | 3/3 | / |
| Negative control | 3.3 10⁸ | 0/2 | 6, 8 |

These data show that intraperitoneal vaccination of Balb/c mice with strain EZ1263 induces protective immunity against *S. enteritidis* phage type 4.

### Example 4 : Induction of protective immunity against S. enteritidis after oral vaccination of mice with S. enteritidis EZ1263

Nine female Balb/c mice, 5 to 6 weeks old, were orally infected with EZ1263 bacteria (by the method described in example 3) in three independent experiments, using a dosage of 1.4 10⁸, 3.4 10⁸ and 1.6 10⁸ colony forming units respectively. All of the infected animals survived without any clear disease symptoms, while all control mice infected with the wild type *S. enteritidis* 76Sa88 under identical conditions were killed by the infection (see Table 4).

**Table 4**

| **Oral infection of mice with wild type** ***S. enteritidis*** **76Sa88 or mutant EZ1263** | | | |
|---|---|---|---|
| Strain | Dose (cfu) | Number of surviving mice | Dead days after inoculation |
| EZ1263* | 3.4 10⁸ | 3/3 | / |
| EZ1263** | 1.3 10⁸ | 3/3 | / |
| EZ1263*** | 1.6 10⁸ | 3/3 | / |
| 76Sa88* | 3.8 10⁸ | 0/3 | 6, 6, 7 |
| 76Sa88** | 2.3 10⁸ | 0/3 | 7, 8, 8 |
| Negative control | 0 | 5/5 | / |
| *, **, ***: Results of three independent experiments | | | |

Six mice that were orally vaccinated with strain EZ1263 were orally challenged with wild type *S*. *enteritidis* 76Sa88 (by the method described in example 3) in two independent experiments . The dose administered was 1.6 10⁸ and 3.3 10⁸ colony forming units respectively. The challenge was carried out 15 respectively 33 days after the oral vaccination with EZ1263. The vaccinated mice survived the challenge infection without showing clear disease symptoms, while all non-vaccinated control mice that were orally challenged under identical conditions were killed (see Table 5).

**Table 5**

| **Protection against oral infection with wild type** ***S. enteritidis*** **76Sa88 after oral infection with mutant EZ1263** | | | |
|---|---|---|---|
| Previous oral infection | Dose of 76Sa88 (cfu) | Number of surviving mice | Dead days after inoculation |
| EZ1263* | 1.6 10⁸ | 3/3 | / |
| none* | 1.6 10⁸ | 0/2 | 8, 9 |
| EZ1263** | 3.3 10⁸ | 3/3 | / |
| none** | 3.3 10⁸ | 0/3 | 9, 10, 11 |
| Negative control | 0 | 1/1 | / |
| *, **: Results of two independent experiments | | | |

These data show that oral vaccination of Balb/c mice with strain EZ1263 induces protective immunity against *S. enteritidis* phage type 4.

### Example 5 : Induction of humoral immunity after oral vaccination of chicks with S. enteritidis EZ1263

Twelve one day old SPF (specific pathogen free) chicks were orally infected with 10⁹ colony forming units of EZ1263 bacteria (cultured for 20 hours in Brain Hearth Infusion broth at 37 °C). Twelve one day old SPF chicks were simultaneously orally infected with 10⁹ colony forming units of the wild type *S. enteritidis* 76Sa88 under identical conditions. Eleven of the 12 chicks infected with EZ1263 survived the infection with minimal disease symptoms and minimal growth retardation. Only 2 in 12 chicks infected with 10⁹ colony forming units of the *S. enteritidis* 76Sa88 survived the infection. These showed severe disease symptoms and growth retardation (see Table 6).

**Table 6**

| **Death and symptoms after oral infection of one day old chicks with wild type** ***S. enteritidis*** **76Sa88 or mutant EZ1263** | | | | | |
|---|---|---|---|---|---|
| Strain | Number of surviving chicks | General depression | Diarrhoea | Growth retardation | Average weight (g) |
| EZ1263 | 11/12 | 0-1 | 2 | 0-1 | 258 ±56 |
| 76Sa88 | 2/12 | 4 | 5 | 3 | 196 ±51 |

The clinical symptoms are represented with a score: 0: no symptoms, 1 to 5: light to very clear symptoms. The average weight of the chick still alive 28 days after infection is given with the standard deviation.

Serum samples of the 11 chicks vaccinated with EZ1263 were taken 4 weeks after infection to test the presence of anti-*Salmonella* antibodies by ELISA essentially as described (Desmidt M., Ducatelle R., Haesebrouck F., de Groot P.A., Verlinden M., Wijffels R., Hinton M., Bale J.A., Allen V.M., Vet. Rec. 138 (10): 223-226, 1996). Microtitre plates (96 wells) were coated with complete *S. enteritidis* (20 hours culture in Brain Hearth Infusion broth at 37 °C, washed in PBS and killed with 99.5 % acetone) or with *S. enteritidis* Lipopolysaccharide (LPS), prepared as described by Westphal O. and Jann K. (in Methods in carbohydrate chemistry, Whistler RL and Wolfrom ML (eds) Academic Press, London p 83-99, 1965). The coating was performed using an antigen solution (10 µg/ml, 150 µl/well) in carbonate/bicarbonate buffer at pH 9.6 for 24 hours at 4 °C. The plates were rinsed once with rinsing buffer (0.05 % Tween 20 in PBS) and blotted on a paper towel. The chick sera were diluted 1:200 in rinsing buffer with 2.2 % skimmed milk powder for the ELISA with the LPS antigen and 1:500 for the ELISA with the complete germ. These diluted sera were incubated on the coated plates for 2 hours at 37 °C. After five rinses, the plates were incubated for 30 min. with rabbit anti chicken immunoglobulin conjugated with horseradish peroxidase (diluted 1:2000 in rinsing buffer with 2.2 % skimmed milk powder). After five rinses, 0.07 % orthophenylene diamine and 0.22 % hydrogen peroxide in citrate buffer were added. After incubation, the reaction was stopped by the addition of 50 µl of 2.5 N HCl. The optical density was determined in a micro-ELISA reader at the a wavelength of 492 nm. The cut-off values for each ELISA were calculated as the mean OD value obtained in ELISA using the sera of 26 (LPS ELISA) or 13 (whole germ ELISA) non-infected control chicks, increased with five times the standard deviation. The experiments were performed *in duplo* and the mean value of the two measurements was calculated. Antibodies directed against *S. enteritidis* phage type 4 LPS were present in 3 out of 11 chicks tested (see Table 7). Using the complete germ as antigen, antibodies could be detected, in the serum of 9 out of 11 chicks (see Table 7). This clearly demonstrates that oral vaccination with EZ1263 induces efficient seroconversion in chicks.

**Table 7**

| **Number of seropositive chicks at 4 weeks post inoculation** | | | | |
|---|---|---|---|---|
| | ELISA using *S. enteritidis* LPS | | ELISA using complete *S*. *enteritidis* | |
| Strain | Number of positive chicks | Total number of chicks | Number of positive chicks | Total number of chicks |
| EZ1263 | 3 | 11 | 9 | 11 |
| 76Sa88 | 1 | 2 | 2 | 2 |

### Example 6 : Transfer of the attenuating mutation of EZ1263 into wild type S. enteritidis and S. typhimurium

The fact that the transposon miniTn*5lacZ1* insertion in EZ1263 was indeed the cause of the attenuation of this strain was established by generalised transduction of the transposon-induced allele into wild type *S. enteritidis* and *S. typhimurium*, using bacteriophage P22HT*int*⁻ (Schmieger H., Phage P22 mutants with increased or decreased transduction abilities. *Mol*.*Gen.Genet*. 119:75-88, 1972). The P22-sensitive virulent bacteria *S. enteritidis* 76Sa88 and *S. typhimurium* 405Sa91, a clinical calf isolate obtained from the Belgian National Veterinary Research Institute (Nationaal Instituut voor Diergeneeskundig Onderzoek), were used as recipients for the transduction.

Transducing bacteriophage stocks were prepared by incubating 10⁴ plaque forming units of bacteriophage P22HT*int*⁻ with 100 µl of an overnight culture of *S. enteritidis* EZ1263 in LB medium at 37 °C for 15 min. Subsequently, 4 ml of top agarose (8 g NaCl, 2 ml 1M MgSO₄ and 6 g agarose per litre) were added and the mixture was poured on top of a fresh LB plate. After overnight incubation at 37 °C, 5 ml of λ buffer (10 mM Tris-HCl pH 7.5; 100 mM NaCl; 10 mM MgCl₂) were added and the plates were gently shaken at room temperature for 2-5 hours to allow the bacteriophages to diffuse. The liquid was subsequently removed with a pipette and 200 µl of chloroform were added. After incubation at 37 °C for 10 min., the suspension was centrifuged (Sorvall SS34 rotor, 15 min, 6000 rpm, 4 °C) and the resulting supernatant was stored at 4 °C in a sterile glass bottle with a few drops of chloroform. This stock was titrated by spotting 20 µl samples of serial dilutions on an LB plate, with a top layer of 100 µl of an overnight broth culture of *S*. *enteritidis* 76Sa88 in top agarose, and counting the number of resulting plaques.

For the transduction, 200 µl of an overnight culture of the recipient bacteria *S. enteritidis* 76Sa88 and *S. typhimurium* 405Sa91 were spun down and resuspended in 80 µl of LB medium. A 10 µl sample of an appropriate dilution of the transducing lysate, giving a multiplicity of infection of below 1, was added and the mixture was incubated for 10-15 min at 37 °C. Subsequently, 4 ml of top agarose was added and the mixture was poured on top of a freshly prepared Petri dish containing two equal layers of culture medium. The bottom LB layer contained 200 mg/l of kanamycin (to select for the presence of the kanamycin resistance gene of the miniTn*5lacZ1*) and the 12,5 ml top layer contained 20 mM EGTA (ethylene glycol-bis(β-amino-ethyl-ether) N,N,N',N'-tetra-acetic acid), a calcium chelating compound preventing further infection by P22HT*int*⁻. After incubation at 37 °C for 24 hours, the resulting kanamycin resistant colonies were purified repeatedly by streaking on LB medium with 100 mg/l kanamycin and 10 mM EGTA. Transductants in *S. enteritidis* 76Sa88 and *S. typhimurium* 405Sa91 were readily obtained using this technique. These data show that the attenuating mutation of strain EZ1263 can be transferred between *Salmonella* strains by standard genetic techniques.

### Example 7 : Attenuated phenotype of a Salmonella typhimurium strain harbouring the attenuating mutation of S. enteritidis EZ1263

To test whether the mutation of *S*. *enteritidis* EZ1263 also induces attenuation in other *Salmonella* serotypes, the virulence of the transductant strain *S. typhimurium* 1263ST405, obtained by transduction of the miniTn*5lacZ1*-generated mutation of *S. enteritidis* EZ1263 into wild type *S. typhimurium* 405Sa91 (see Example 6), was tested. Oral infection of Balb/c mice with about 1.9 10⁸ colony forming units (cfu) of *S. typhimurium* 1263ST405 was performed as described previously (see Example 4). The morbidity and mortality data (see Table 8) indicate that the attenuated phenotype of *S. enteritidis* EZ1263 is linked to the transposon insertion. In addition, the results prove that the gene that is inactivated in *S*. *enteritidis* EZ1263 is also required for the virulence of *S. typhimurium.*

**Table 8**

| **Oral infection of mice with a *S. typhimurium* strain harbouring the attenuating mutation of** ***S. enteritidis*** **EZ1263** | | | |
|---|---|---|---|
| Strain | Dose (cfu) | Number of surviving mice | Dead days after inoculation |
| *S. typhimurium* 1263ST405 | 1.89 10⁸ | 3/3 | / |
| *S. typhimurium* 405Sa91 (wild type) | 2.44 10⁸ | 0/3 | 7, 8, 10 |
| Negative control | 0 | 5/5 | / |

### Example 8 : Induction of humoral immunity against both S. enteritidis and F17 fimbriae after oral vaccination of mice with S. enteritidis EZ1263 harbouring a plasmid encoding the production of F17 fimbriae

To test whether *S. enteritidis* EZ1263 can be used as a carrier for foreign antigens in the production of recombinant live vaccines, the plasmid pPLHD54 (Lintermans P., Karakterisatie van de F17 en F111 fimbriae van *Escherichia coli* en genetische analyse van de F17 genkluster, Proefschrift tot het verkrijgen van de graad van geaggregeerde van het hoger onderwijs, RUG, 1990), encoding the production of F17 fimbriae, was introduced into *S. enteritidis* EZ1263.

To avoid excessive restriction, the plasmid was first introduced by electroporation (O'Callaghan D. and Charbit A., Mol Gen Genet 223: 156-158, 1990) into a *S*. *typhimurium hsd* mutant (Nakayama K., Kelly S.M., Curtiss III R., Bio/Technology 6:693-697, 1988). Plasmid DNA was subsequently prepared from a transformant (JETstar 2.0 Plasmid MIDI Kit, Genomed) and used to electroporate EZ1263.

To test the immunogenicity of the resulting strain *S. enteritidis* EZ1263 (pPLHD54), 5-6 weeks old female Balb/c mice were orally vaccinated with about 10⁸ colony forming units per mouse as described previously (see Example 4). The vaccination was repeated after 3 weeks.

Blood samples were collected before the first immunisation, at different times after the first infection and at 12 days after the second infection. The serum was separated by incubation of the sample for 1 hour at 37 °C followed by incubation for 2 hours at 4 °C and two centrifugations at 12.000 rpm in an Eppendorf micro-centrifuge and stored at -20 °C.

Microtitre plates (96 wells) were coated with F17 fimbriae or *S. enteritidis* LPS (Sigma Chemie, lyophilised powder prepared by phenol extraction) using an antigen solution (2 µg/ml, 100 µl/well) in PBS for 1 hour at 37 °C. The plates were rinsed three times with PBS containing 1% Tween 80. Subsequently, 200 µl per well of a 5 mg/ml solution of bovine serum albumin (BSA) in PBS were added and the plates were incubated at 37 °C for 30 min. The plates were rinsed again three times with PBS containing 1% Tween 80.

The sera were diluted (1:100, 1:300, 1:900 after vaccination and 1:10 and 1:100 for the preimmune sera) in PBS. After addition of 50 µl of serum in each well, the plates were incubated for 1 hour at 37 °C and rinsed 6 times with PBS containing 1% Tween 80. Subsequently, 100 µl of a 1:1000 dilution of the goat anti mouse immunoglobulin conjugated with horseradish peroxidase were added. The plates were incubated for 1 hour at room temperature and washed 6 times with PBS containing 11% Tween 80. Subsequently, 100 µl substrate solution (TMB Peroxidase EIA Substrate Kit, Bio-Rad) were added in each well. The reaction was stopped after 15 min by the addition of 100 µl 1 M H₃PO₄. The optical density was determined in a micro-ELISA reader at the wavelength of 450 nm. The cut-off value for each ELISA was 2.5 times the OD value of the pre-immune serum.

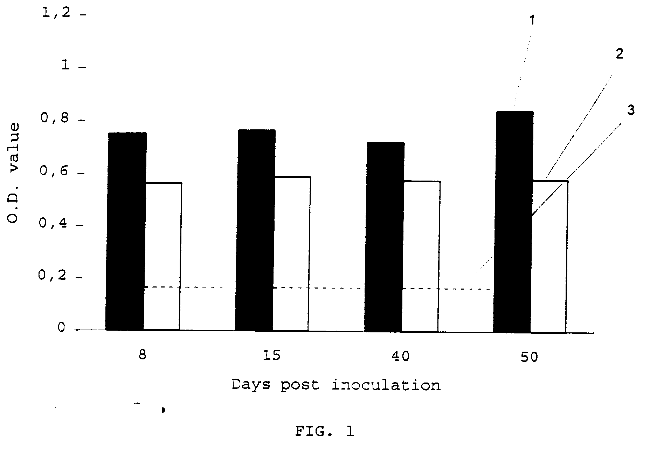

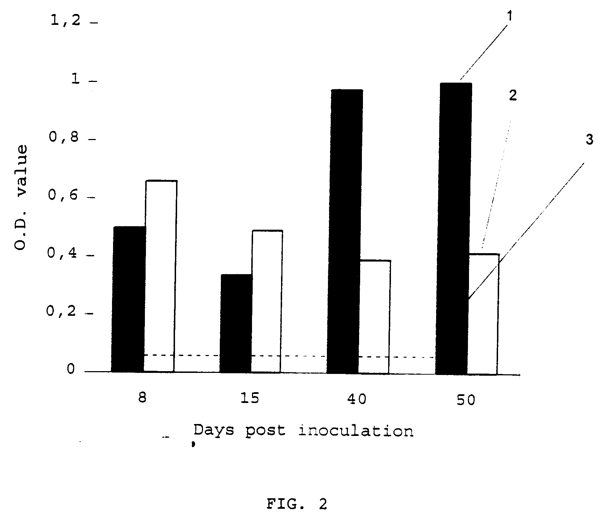

The results of the ELISA test showed that antibodies directed against F17 fimbriae (Figure 1) and against *S. enteritidis* LPS (see Figure 2) were present in both of the tested mice (1 = mouse 1; 2 = mouse 2), and are clearly above the cut-off (3). The antibody titre remained high for at least 50 days. This clearly demonstrates that oral vaccination with EZ1263 expressing F17 fimbriae induces the production of antibodies directed against both *Salmonella* LPS and F17 fimbriae. EZ1263 can therefore be used as a carrier for the expression of foreign epitopes.

### Example 9 : Conversion of the transposon insertion mutation of S. enteritidis EZ1263 into a deletion

To avoid any reversion of the attenuating mutation present in EZ1263 and to remove the transposon sequence with its kanamycin resistance gene from this strain, a deletion of the relevant sequence will be introduced by homologous recombination. The 7-8 kb *Bam*HI fragment hybridising with the EZ1263-probe (see example 2) was cloned in the *Xho*I site of pACYC177 after partial fill-in of the *Bam*H1 and *Xho*I sticky ends. This resulted in the plasmid pGV4484. An internal *Pst*I deletion, removing the 1.6 kb *Pst*I fragment in which the minitransposon is inserted in strain EZ1263, was introduced in the *Bam*HI insert of this plasmid by *Pst*I digestion followed by self-ligation. This resulted in the deletion of the complete open reading frame that was interrupted by the transposon insertion.

The fragment carrying the *Pst*I deletion will be ligated into a suitable site in the suicide vector pUT (Herrero M., de Lorenzo V., Timmis K.N., J. Bacteriol. 172 (11): 6557-6567, 1990) , that is unable to replicate autonomously in *Salmonella*, and transformed in strain *E*. *coli* S17-1(λpir). The suicide plasmid, carrying the deletion, will be mobilised to *Salmonella* strain EZ1263 or 1263SEWT (a rifampicin sensitive strain obtained by P22-mediated transduction of the transposon insertion of EZ1263 into wild type S. enteritidis 76Sa88, as described in Example 6). The mobilisation will be performed by overnight incubation of a mixture of 100 µl of the donor and recipient strains. The integration of the suicide vector into the *Salmonella* genome, by a single recombination between homologous sequences, will be selected on LB medium containing 100 µg/l rifampicin, to counter-select the donor *E. coli* strain S17-1(λpir), and 100 µg/ml carbenicillin (marker of the suicide plasmid pUT) when EZ1263 is used as a recipient or on Minimal A medium (Miller, J.H. *Experiments in Molecular Genetics*, Cold Spring Harbor, New York: Cold Spring Harbor Laboratory, 1972. pp. 1-466) containing 100 µg/ml carbenicillin when 1263SEWT is the recipient. After colony purification on selective medium, several transconjugant *Salmonella* strains will be grown in liquid LB medium without antibiotics and plated on LB medium without antibiotics. The resulting colonies (about 500 per Petri dish) will be replica plated on LB medium supplemented with carbenicillin (100 µg/ml), on LB medium supplemented with kanamycin (50 µg/ml) and on LB medium without antibiotics. Double recombinants will be identified as sensitive to the antibiotics carbenicillin and kanamycin. The presence of the deletion is subsequently confirmed by Southern DNA hybridisation using the deleted 1.6 kb *Pst*I fragment as a probe.

### Example 10 : Presence of the DNA sequence that is mutated in EZ1263 in other bacteria

The presence of the gene that is mutated in EZ1263 in the genome of various *Salmonella* strains and other *Enterobacteriaceae* was investigated by two different strategies: DNA hybridisation and PCR analysis with specific primers. Except where mentioned, the experiments were performed using standard procedures (Sambrook J., Fritsch E.F., Maniatis T., Molecular cloning, a laboratory manual, Second edition, Cold Spring Harbor Laboratory Press, Cold Spring Harbor, 1989). Total genomic DNA of the relevant bacterial cultures, all obtained from the collection of the Belgian National Institute of Veterinary Research, was isolated as described in Example 2.

### i. Southern DNA hybridisation

After total digestion by the restriction endonucleases *Eco*RI or *Hin*dIII, 4 µg of total genomic DNA was separated on a 0.8 % agarose gel and transferred onto a nylon membrane (Hybond-N, Amersham) as indicated by the supplier ("Blotting and hybridisation protocols for Hybond™ Membranes, Amersham).

The filter was pre-hybridised for 2 hours at 65 °C in a solution consisting of 5.8 ml H₂0, 3 ml 20×SSC (3 M NaCl, 0.3 M Na₃-Citrate), 0.5 ml 100 x Denhardt's solution (2%[w/v]BSA, 2% [w/v] Ficoll™ and 2% polyvinyl-pyrollidone), 0.5 ml 10% SDS and 0.2 ml denatured herring sperm DNA (1 mg/ml).

The probe was prepared by radio-labelling 25 ng of the IPCR fragment of the mutant EZ1263 with [α-³²P]dCTP using the Amersham RPN 1601Y Multiprime DNA Labelling Kit. The labelled DNA was separated from the free nucleotides using a Sephadex PD-10 G-25M column. The column was first equilibrated twice with 5 ml buffer (10 mM Tris, 100 mM NaCl, 1 mM EDTA, pH8). The labelled DNA was eluted using the same buffer and the most radio-active fractions were pooled.

The labelled probe was denatured (5 min at 90 °C), cooled on ice for 10 min. and added to the pre-hybridised filter. The filter was incubated overnight at 65 °C. The non-hybridised probe was removed by washing as described in the Amersham protocol. The signal was detected by putting a Fuji X-ray film on top of the membrane and incubating at -70 °C, for 2-5 hours or overnight.

### ii. PCR analysis

The genomic DNA was diluted 1:50 and used for a PCR amplification using the following reaction mixture: 10 µl diluted genomic DNA, 1 µl 20 µM primer 1263-1 (Table 1), 1 µl 20 µM primer 1263-2B (Table 1), 4 µl of a solution containing 2.5 mM of each dNTP, 5 µl SuperTaq buffer and 0.1 µl SuperTaq in a total volume of 50 µl. The PCR reaction consisted of 25 cycles of 94 °C for 10 sec, 55 °C for 20 sec and 72 °C for 90 sec. The PCR products were detected on a 0.8% agarose gel.

### iii. Results

Using hybridisation as well as PCR, comparable results were obtained. The data, presented in Table 9, show that nucleotide sequences homologous with SEQ ID 01 are detected only in *Salmonella* strains. This indicates that the *Salmonella* mutant EZ1263 carries a mutation in a virulence gene that is specific for *Salmonella*.

**Table 9**

| **Detection of the DNA sequence that is mutated in EZ1263 in other bacteria** | | | | |
|---|---|---|---|---|
| Species or serotype | Strain | DNA hybridisation | | PCR |
| | | EcoRI | HindIII | |
| *Aeromonas hydrophilla* | 2663 | - | - | - |
| *Aeromonas hydrophilla* | 2717 | - | - | - |
| *Bordetella bronchiseptica* | 2790S25 | - | - | - |
| *Citrobacter anomaloniticus* | 2688 | - | - | - |
| *Citrobacter anomaloniticus* | 2512 | - | - | - |
| *Enterobacter cloacae* | 2811S | - | - | ND |
| *Escherichia coli* O:1 | Ørskov *et al*., 1977¹ | *-* | *-* | *-* |
| *Escherichia coli* O:86 | Ørskov *et al*., 1977¹ | - | - | - |
| *Pasteurella haemolytica* | 2589S | - | - | - |
| *Plesiomonas shigelloides* | 2716 | - | - | - |
| *Proteus mirabilis* | 256ani | - | - | ND |
| *Salmonella enteritidis* | 76Sa88 | + | + | + |
| *Salmonella hadar* | 373Sa95 | + | + | + |
| *Salmonella infantis* | 642Sa95 | ND | ND | + |
| *Salmonella montevideo* | 480Sa95 | ND | ND | + |
| *Salmonella senftenberg* | 402Sa95 | ND | ND | + |
| *Salmonella typhimurium* | 405Sa91 | + | + | + |
| ND = not done | | | | |

| | | | | |
|---|---|---|---|---|
| ¹ Ørskov I., Ørskov F., Jann B., Jann K., Bacteriol. Rev. 41:667-710, 1977 | | | | |

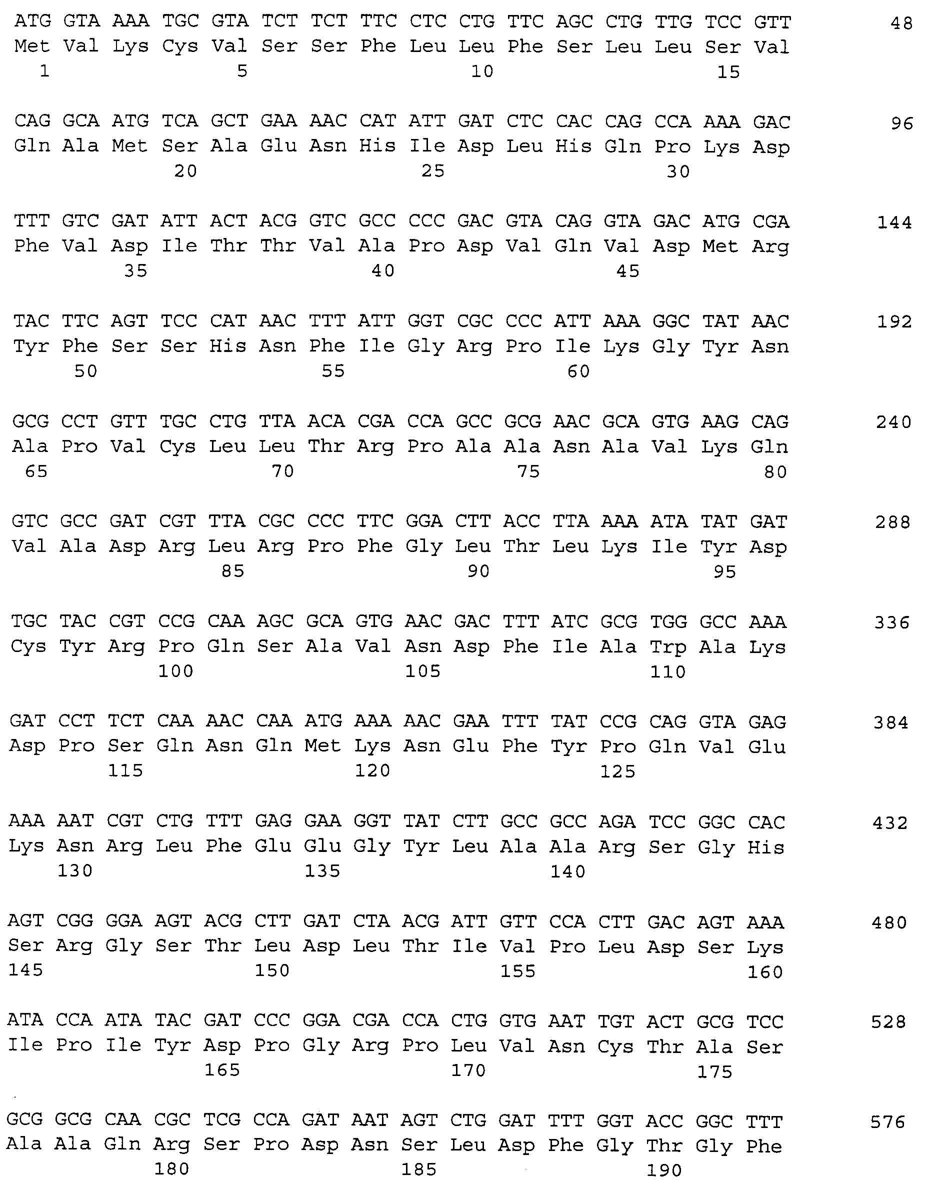

## Claims

1. A vaccine for inducing an immune response to a *Salmonella* strain in an animal, including a human, characterised in that it comprises a pharmaceutically acceptable carrier and a genetically modified *Salmonella* strain which is in an amount effective to produce an immune response in said animal, including human, and comprises a modification in its wild type DNA sequence SEQ ID NO 01, any of the DNA sequences from the same operon as SEQ ID NO 01 and/or any regulatory sequences of any of the said DNA sequences.

2. A vaccine according to claim 1, characterised in that the genetically modified *Salmonella* strain contains an isolated *Salmonella*-foreign nucleotide sequence encoding a *Salmonella*-foreign antigen and in that said genetically modified *Salmonella* strain is in an amount effective to produce an immune response to said *Salmonella-*foreign antigen in said animal, including human.

3. A vaccine according to claim 1 or 2, characterised in that the modification in the DNA sequence SEQ ID NO 01 is an insertion or a deletion of at least one nucleotide in the wild type DNA sequence SEQ ID NO 01.

4. A vaccine according to any of the preceding claims, characterised in that the *Salmonella* strain is selected from the group consisting of: *Salmonella enteritidis, Salmonella typhimurium, Salmonella choleraesuis, Salmonella dublin, Salmonella paratyphi, Salmonella typhi, Salmonella hadar, Salmonella infantis, Salmonella montevideo* and *Salmonella senftenberg.*

5. A vaccine according to claim 4, characterised in that the *Salmonella* strain is the *Salmonella enteritidis* EZ1263 having the deposit number LMPG-18112

6. An isolated or synthetic virulent DNA sequence, characterised in that it has at least 40% homology with the wild type DNA sequence SEQ ID NO 01 or its complementary strand.

7. An isolated or synthetic virulent DNA sequence as in claim 6, characterised in that it has at least 55 % homology with the wild type DNA sequence SEQ ID NO 01 or its complementary strand.

8. An isolated or synthetic virulent DNA sequence as in claim 6, characterised in that it has at least 70 % homology with the wild type DNA sequence SEQ ID NO 01 or its complementary strand.

9. An isolated or synthetic virulent DNA sequence as in claim 6, characterised in that it has at least 85 % homology with the wild type DNA sequence SEQ ID NO 01 or its complementary strand.

10. An isolated or synthetic virulent DNA sequence as in claim 6, characterised in that it is homologous with the wild type DNA sequence SEQ ID NO 01 or its complementary strand.

11. An isolated or synthetic virulent amino acid sequence, possibly encoded by the virulent DNA sequence according to any of the claims 6 to 10, and that has at least 30 % homology with the wild type amino acid sequence SEQ ID NO 02.

12. An isolated or synthetic virulent amino acid sequence such as in claim 11, characterised in that it has at least 50 % homology with the wild type amino acid sequence SEQ ID NO 02.

13. An isolated or synthetic virulent amino acid sequence such as in claim 11, characterised in that it has at least 75 % homology with the wild type amino acid sequence SEQ ID NO 02.

14. An isolated or synthetic virulent amino acid sequence such as in claim 11, characterised in that it has at least 90 % homology with the wild type amino acid sequence SEQ ID NO 02.

15. An isolated or synthetic virulent amino acid sequence such as in claim 11, characterised in that it is homologous with the wild type amino acid sequence SEQ ID NO 02.

16. Preparation method of an avirulent *Salmonella* strain, comprising the steps of :
- identifying a "virulent" nucleotide sequence in the genome of a *Salmonella* strain by any method based on the use of nucleotide sequence SEQ ID NO 01 or the complementary strand thereof, such as hybridisation or amplification by the polymerase chain reaction with a probe or primers having at least 12 nucleotides and which shows at least 10 identical nucleotides with a corresponding portion of SEQ ID NO 01 or its complementary strand or which shows more than 50% homology with a corresponding portion of SEQ ID NO 01 or its complementary strand.
- inducing a modification in said "virulent" nucleotide sequence, and
- recovering an obtained avirulent *Salmonella* strain having said modification in its "virulent" sequence.

17. The method according to claim 16, characterised in that the modification in said virulent sequence is an insertion or a deletion of at least one nucleotide in said "virulent" sequence.

18. A method for inducing an immune response to a *Salmonella* strain in an animal, including a human, comprising administering a live, genetically modified *Salmonella* strain to said animal, including human, wherein said genetically modified *Salmonella* strain is in an amount effective to produce an immune response.

19. The method according to claim 18, wherein said genetically modified *Salmonella* strain is administered in a pharmaceutically acceptable carrier.

20. Use of the vaccine according to any of the preceding claims 1 to 5 for the manufacture of a medicament for inducing an immune response to a *Salmonella* strain in an animal, including a human.

21. Use according to claim 20, characterised in that the immune response to the *Salmonella* strain in the animal, including the human, is a humoral, local and/or cellular immune response.


SEQUENCE LISTING

(1) GENERAL INFORMATION:

(i) APPLICANT:
(A) NAME: Vrije Universiteit Brussel
(B) STREET: Pleinlaan 2
(C) CITY: Brussels
(E) COUNTRY: Belgium
(F) POSTAL CODE (ZIP): B-1050

(ii) TITLE OF INVENTION: LIVE ATTENUATED SALMONELLA VACCINE

(iii) NUMBER OF SEQUENCES: 8

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 771 base pairs
(B) TYPE: nucleic acid
(C) STRANDEDNESS: single
(D) TOPOLOGY: linear

(ii) MOLECULE TYPE: peptide

(ix) FEATURE:
(A) NAME/KEY: primer_bind
(B) LOCATION:380..397

(ix) FEATURE:
(A) NAME/KEY: primer_bind
(B) LOCATION:complement (172..189)

(ix) FEATURE:
(A) NAME/KEY: CDS
(B) LOCATION:1..771

(xi) SEQUENCE DESCRIPTION: SEQ ID NO: 1:

GAC TGT TTT AGT CCG TTA TCC CAT CCC GAT AAT GTC ATG CTT ACC GCT 624
Asp Cys Phe Ser Pro Leu Ser His Pro Asp Asn Val Met Leu Thr Ala
195 200 205

CAG CAA CGC GCA AAT AGG TTG TTA TTA CAA ACA TTG ATG CGT GAT GCG 672
Gln Gln Arg Ala Asn Arg Leu Leu Leu Gln Thr Leu Met Arg Asp Ala
210 215 220

GGT TTT ACG CCA CTG GAT ACT GAA TGG TGG CAC TTC TCT CTT ACT CAT 720
Gly Phe Thr Pro Leu Asp Thr Glu Trp Trp His Phe Ser Leu Thr His
225 230 235 240

GAA CCA TAC CCG AAC ACT TGG TTT GAC TTT CCC GTT AAG CAG AGA CCC 768
Glu Pro Tyr Pro Asn Thr Trp Phe Asp Phe Pro Val Lys Gln Arg Pro
245 250 255

TGA 771
*

(2) INFORMATION FOR SEQ ID NO: 2:

(i) SEQUENCE CHARACTERISTICS:
(A) LENGTH: 257 amino acids
(B) TYPE: amino acid
(D) TOPOLOGY: linear

(ii) MOLECULE TYPE: protein
(xi) SEQUENCE DESCRIPTION: SEQ ID NO: 2:

Met Val Lys Cys Val Ser Ser Phe Leu Leu Phe Ser Leu Leu Ser Val
1               5                   10                  15

Gln Ala Met Ser Ala Glu Asn His Ile Asp Leu His Gln Pro Lys Asp
            20                  25                  30

Phe Val Asp Ile Thr Thr Val Ala Pro Asp Val Gln Val Asp Met Arg
        35                  40                  45

Tyr Phe Ser Ser His Asn Phe Ile Gly Arg Pro Ile Lys Gly Tyr Asn
    50                  55                  60

Ala Pro Val Cys Leu Leu Thr Arg Pro Ala Ala Asn Ala Val Lys Gln
65                  70                  75                  80

Val Ala Asp Arg Leu Arg Pro Phe Gly Leu Thr Leu Lys Ile Tyr Asp

                85                  90                  95

Cys Tyr Arg Pro Gln Ser Ala Val Asn Asp Phe Ile Ala Trp Ala Lys
            100                 105                 110

Asp Pro Ser Gln Asn Gln Met Lys Asn Glu Phe Tyr Pro Gln Val Glu
        115                 120                 125

Lys Asn Arg Leu Phe Glu Glu Gly Tyr Leu Ala Ala Arg Ser Gly His
    130                 135                 140

Ser Arg Gly Ser Thr Leu Asp Leu Thr Ile Val Pro Leu Asp Ser Lys
145                 150                 155                 160

Ile Pro Ile Tyr Asp Pro Gly Arg Pro Leu Val Asn Cys Thr Ala Ser
                165                 170                 175

Ala Ala Gln Arg Ser Pro Asp Asn Ser Leu Asp Phe Gly Thr Gly Phe
            180                 185                 190

Asp Cys Phe Ser Pro Leu Ser His Pro Asp Asn Val Met Leu Thr Ala
        195                 200                 205

Gln Gln Arg Ala Asn Arg Leu Leu Leu Gln Thr Leu Met Arg Asp Ala
    210                 215                 220

Gly Phe Thr Pro Leu Asp Thr Glu Trp Trp His Phe Ser Leu Thr His
225                 230                 235                 240

Glu Pro Tyr Pro Asn Thr Trp Phe Asp Phe Pro Val Lys Gln Arg Pro
                245                 250                 255

*

(2) INFORMATION FOR SEQ ID NO: 3:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 3:

GGGAATTCAA AGCGCCATTC GCCATTCAG 29

(2) INFORMATION FOR SEQ ID NO: 4:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 4:

GGAAGCTTTA TGGCAGGGTG AAACGCAGG 29

(2) INFORMATION FOR SEQ ID NO: 5:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 5:

CGTCTAGACG TTTTCCCAGT CACGAC 26

(2) INFORMATION FOR SEQ ID NO: 6:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 6:

GCGGATCCTT TCGGCGGTGA AATTATC 27

(2) INFORMATION FOR SEQ ID NO: 7:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 7:

ACAGACGATT TTTCTCTA 18

(2) INFORMATION FOR SEQ ID NO: 8:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 8:

CGCCCCATTA AAGGCTAT 18